# EUROPÄISCHE PATENTANMELDUNG

(11) **EP 4 250 307 A1**
(43) Veröffentlichungstag der Anmeldung: **27.09.2023**
(21) Anmeldenummer: 22192654.6
(22) Anmeldetag: 29.08.2022
(51) Int. Cl.: G16H 10/60, G16H 20/40, G16H 40/63

(54) **VERFAHREN UND DATENVERARBEITUNGSSYSTEM ZUM BEREITSTELLEN EINER STUDIENBESCHREIBUNG FÜR EINE RADIOLOGISCHE BILDGEBUNGSUNTERSUCHUNG**

(71) Anmelder: Siemens Healthcare GmbH, 91052 Erlangen (DE)
(72) Erfinder: Allmendinger, Thomas, 91301 Forchheim (DE); Feuerlein, Ute, 91052 Erlangen (DE)

(57) **Zusammenfassung**

Die Erfindung betrifft ein computerimplementiertes Verfahren zum Bereitstellen einer Studienbeschreibung für eine radiologische Bildgebungsuntersuchung, das Verfahren umfassend:
- ein Empfangen eines Konfigurationsdatensatzes, welcher eine Konfiguration eines Protokolls für die radiologische Bildgebungsuntersuchung betrifft,
- ein Empfangen von Zuordnungsdaten, wobei jeder Konfigurationsbeschreibung einer Mehrzahl von Konfigurationsbeschreibungen mittels der Zuordnungsdaten jeweils eine Studienbeschreibung aus einer Mehrzahl von Studienbeschreibungen zugeordnet ist,
- ein Ermitteln einer relevanten Konfigurationsbeschreibung aus der Mehrzahl von Konfigurationsbeschreibungen basierend auf dem Konfigurationsdatensatz und den Zuordnungsdaten,
- ein Bereitstellen derjenigen Studienbeschreibung der Mehrzahl von Studienbeschreibungen, welche mittels der Zuordnungsdaten der relevanten Konfigurationsbeschreibung zugeordnet ist.

## Beschreibung

Die Erfindung betrifft ein Verfahren zum Bereitstellen einer Studienbeschreibung für eine radiologische Bildgebungsuntersuchung. Die Erfindung betrifft ferner ein Datenverarbeitungssystem, ein medizinisches Bildgebungsgerät, ein Computerprogrammprodukt und ein computerlesbares Medium.

Um eine radiologische Bildgebungsuntersuchung mittels eines entsprechend eingerichteten medizinischen Bildgebungsgeräts durchführen zu können, werden technische Parameter für einen oder mehrere Scans zur Bildgebungsdatenakquisition basierend auf einem Protokoll für die radiologische Bildgebungsuntersuchung konkretisiert. Als Ausganspunkt für die Auswahl des Protokoll kann insbesondere eine klinische Anforderung (Requested Procedure) dienen, welche beispielsweise von einem Radiologie-Informationssystem (RIS) an das medizinische Bildgebungsgerät gesendet werden kann, insbesondere über eine DICOM Modality Worklist (DMWL) gesendet werden kann.

Zum Speichern und Verarbeiten der auf die radiologische Bildgebungsuntersuchung bezogenen Daten wir dieser eine Studienbeschreibung zugeordnet. Die Studienbeschreibung kann beispielsweise verwendet werden, um der radiologischen Bildgebungsuntersuchung Abrechnungscodes, Befunder, PACS-Daten-Darstellungen (hanging protocols) und/oder Dosisauswertungsparameter (z. B. im Rahmen eines Lungscreenings) zuzuordnen. Darüber hinaus müssen die Protokolle in bestimmten Fällen in der Software für eine Dosisberichterstattung spezifisch benannt werden.

Grundsätzlich ist es möglich, den Namen des Protokolls oder die klinische Anforderung als Studienbeschreibung zu verwenden. Durch die Möglichkeit, das Protokoll für die radiologisehe Bildgebungsuntersuchung zu konfigurieren, insbesondere in Abhängigkeit von Patientendaten zu konfigurieren, ist die Indikation jedoch nicht mehr ohne Weiteres eindeutig einer Studienbeschreibung zuordenbar. So kann z. B. aus einem nativen Kopfscan ad hoc eine Perfusionsauswertung mit nachfolgender Carotis-CTA werden. Insbesondere wenn sich während der Bildgebungsuntersuchung die ursprüngliche Indikation ändert, beispielsweise wenn eine ursprünglich indizierte Hirnperfusion aufgrund einer während der Bildgebungsuntersuchung festgestellten Hirnblutung nicht durchgeführt wird, kann es zu Unstimmigkeiten zwischen einer an der ursprünglichen Indikation orientierten Studienbeschreibung und der tatsächlich durchgeführten Bildgebungsuntersuchung kommen.

DE 10 2018 216 740 B4 offenbart ein Verfahren zum Übermitteln von patientenspezifischen Daten an eine Untersuchungsprotokollanpassungseinheit zur Anpassung eines Untersuchungsprotokolls für eine medizinische Bildgebungsuntersuchung eines Patienten basierend auf den patientenspezifischen Daten.

Die Erfindung hat die Aufgabe, eine verbesserte Zuordnung von Studienbeschreibungen für radiologische Bildgebungsuntersuchungen zu ermöglichen.

Die Erfindung betrifft ein computerimplementiertes Verfahren zum Bereitstellen einer Studienbeschreibung für eine radiologische Bildgebungsuntersuchung, das Verfahren umfassend:
- ein Empfangen eines Konfigurationsdatensatzes, welcher eine Konfiguration eines Protokolls für die radiologische Bildgebungsuntersuchung betrifft,
- ein Empfangen von Zuordnungsdaten, wobei jeder Konfigurationsbeschreibung einer Mehrzahl von Konfigurationsbeschreibungen mittels der Zuordnungsdaten jeweils eine Studienbeschreibung aus einer Mehrzahl von Studienbeschreibungen zugeordnet ist, insbesondere eindeutig zugeordnet ist,
- ein Ermitteln einer relevanten Konfigurationsbeschreibung aus der Mehrzahl von Konfigurationsbeschreibungen basierend auf dem Konfigurationsdatensatz und den Zuordnungsdaten,
- ein Bereitstellen derjenigen Studienbeschreibung der Mehrzahl von Studienbeschreibungen, welche mittels der Zuordnungsdaten der relevanten Konfigurationsbeschreibung zugeordnet ist.

Insbesondere kann das Protokoll derart angelegt sein, dass es grundsätzlich für verschiedene Indikationen verwendet werden kann. Beispielsweise kann das Protokoll ein Kopfbildgebungsprotokoll sein, das sowohl für eine Hirnblutung als auch für eine Hirnperfusion verwendet werden kann.

Die radiologische Bildgebungsuntersuchung kann insbesondere eine medizinisch-radiologische Bildgebungsuntersuchung sein. Das Protokoll kann beispielsweise Werte für technische Parameter der radiologischen Bildgebungsuntersuchung, beispielsweise für eine Röntgenröhrenspannung, explizit aufweisen. Alternativ oder zusätzlich kann das Protokoll beispielsweise Werte für klinische Parameter der radiologischen Bildgebungsuntersuchung, beispielsweise für eine Körperform eines Untersuchungsobjekts, aufweisen, aus denen Werte für diejenigen technischen Parameter der radiologischen Bildgebungsuntersuchung, die im Protokoll nicht explizit enthalten sind, abgeleitet und/oder berechnet werden können.

Die Konfiguration des Protokolls für die radiologische Bildgebungsuntersuchung kann beispielsweise eine Optimierung des Protokolls basierend auf dem Konfigurationsdatensatz umfassen. Die tatsächlich durchzuführende radiologische Bildgebungsuntersuchung ist durch das konfigurierte Protokoll hinreichend spezifiziert. Durch die Konfiguration des Protokolls kann die radiologische Bildgebungsuntersuchung beispielsweise in Bezug auf eine ausgewählte Indikation angepasst sein, insbesondere optimiert sein. Auf diese Weise kann die Anzahl von zu verwaltenden spezifischeren Protokollen verringert werden.

Insbesondere kann vorgesehen sein, dass die Konfiguration des Protokolls für die radiologische Bildgebungsuntersuchung basierend auf dem Konfigurationsdatensatz durchgeführt wird.

Insbesondere kann vorgesehen sein, dass die radiologische Bildgebungsuntersuchung basierend auf der Konfiguration des Protokolls durchgeführt wird, beispielsweise mittels eines medizinischen Bildgebungsgeräts durchgeführt wird, um radiologische Bildgebungsdaten eines Untersuchungsobjekts zu erfassen.

Weiterhin kann vorgesehen sein, dass die radiologischen Bildgebungsdaten mit der Studienbeschreibung verknüpft werden, beispielsweise indem ein Bildgebungsdatenpaket basierend auf den radiologischen Bildgebungsdaten und der Studienbeschreibung generiert wird. Beispielsweise kann das Bildgebungsdatenpaket Nutzdaten, welche die radiologischen Bildgebungsdaten umfassen, und Kopfdaten, welche die Studienbeschreibung umfassen, aufweisen. Weiterhin kann vorgesehen sein, dass das Bildgebungsdatenpaket bereitgestellt wird.

Durch die flexible Zuordnung von Studienbeschreibungen in Abhängigkeit von der tatsächlich durchgeführten Bildgebungsuntersuchung kann der Aufwand für die Protokollpflege deutlich verringert werden. Zudem kann eine Befundungszeit deutlich verringert werden, da über die verbesserte Zuordnung der Studienbeschreibung sichergestellt werden kann, dass die Daten, welche durch die radiologische Bildgebungsuntersuchung gewonnen wurden, einer passenden Befundungsstelle in einer korrekten Darstellung zugewiesen werden. Ferner kann damit eine Genauigkeit einer klinischen Kostenabrechnung verbessert werden und/oder eine auf der Studienbeschreibung basierende automatisierte Abrechnung realisiert werden. Außerdem kann damit eine Genauigkeit von auf der Studienbeschreibung basierenden Auswertungen, insbesondere statistischen Auswertungen, welche beispielsweise eine Dosisberichterstattung und/oder klinische Leistungskennzahlen betreffen, verbessert werden.

Die Zuordnungsdaten können beispielsweise die Mehrzahl von Konfigurationsbeschreibungen und/oder die Mehrzahl von Studienbeschreibungen umfassen. Weiterhin kann vorgesehen sein, dass die Zuordnungsdaten eine Default-Studienbeschreibung umfassen, welche nicht in der Mehrzahl von Studienbeschreibungen enthalten ist und/oder welche keiner Konfigurationsbeschreibung der Mehrzahl von Konfigurationsbeschreibungen zugeordnet ist. Die Default-Studienbeschreibung kann beispielsweise in Fällen verwendet werden, in denen der Konfigurationsdatensatz mit keiner der Konfigurationsbeschreibungen der Mehrzahl von Konfigurationsbeschreibungen kompatibel ist.

Weiterhin kann vorgesehen sein, dass die Zuordnungsdaten eine Konfigurationsdatenstruktur aufweisen und/oder dass die relevante Konfigurationsbeschreibung aus der Mehrzahl von Konfigurationsbeschreibungen basierend auf dem Konfigurationsdatensatz und der Konfigurationsdatenstruktur der Zuordnungsdaten ermittelt wird. Die Konfigurationsdatenstruktur kann insbesondere auf einer Mehrzahl von Konfigurationsstrategien und/oder auf einer Definition von Konfigurationsvariantenmengen basieren. Insbesondere können der Konfigurationsdatensatz und die Konfigurationsdatenstruktur zueinander kompatibel sein.

Eine Ausführungsform sieht vor, dass der Konfigurationsdatensatz für jede Konfigurationsstrategie einer Mehrzahl von Konfigurationsstrategien jeweils einen Konfigurations-Aktivitätswert, welcher einen Aktivitätsstatus dieser Konfigurationsstrategie bei der Konfiguration des Protokolls für die radiologische Bildgebungsuntersuchung anzeigt, aufweist,
- wobei jede Konfigurationsbeschreibung der Mehrzahl von Konfigurationsbeschreibungen für jede Konfigurationsstrategie der Mehrzahl von Konfigurationsstrategien jeweils einen Beschreibungs-Aktivitätswert aufweist,
- wobei das Ermitteln der relevanten Konfigurationsbeschreibung aus der Mehrzahl von Konfigurationsbeschreibungen für wenigstens eine Konfigurationsstrategie der Mehrzahl von Konfigurationsstrategien einen Vergleich, insbesondere einen Identitätsvergleich, des jeweiligen Konfigurations-Aktivitätswerts des Konfigurationsdatensatzes mit dem jeweiligen Beschreibungs-Aktivitätswert einer ersten Konfigurationsbeschreibung aus der Mehrzahl von Konfigurationsbeschreibungen umfasst.

Insbesondere kann vorgesehen sein, dass das Ermitteln der relevanten Konfigurationsbeschreibung aus der Mehrzahl von Konfigurationsbeschreibungen für jede Konfigurationsstrategie der Mehrzahl von Konfigurationsstrategien einen Vergleich, insbesondere einen Identitätsvergleich, des jeweiligen Konfigurations-Aktivitätswerts des Konfigurationsdatensatzes mit dem jeweiligen Beschreibungs-Aktivitätswert einer ersten Konfigurationsbeschreibung aus der Mehrzahl von Konfigurationsbeschreibungen umfasst.

Insbesondere kann vorgesehen sein, dass das Ermitteln der relevanten Konfigurationsbeschreibung aus der Mehrzahl von Konfigurationsbeschreibungen für jede Konfigurationsstrategie der Mehrzahl von Konfigurationsstrategien und für jede Konfigurationsbeschreibung aus der Mehrzahl von Konfigurationsbeschreibungen einen Vergleich, insbesondere einen Identitätsvergleich, des jeweiligen Konfigurations-Aktivitätswerts des Konfigurationsdatensatzes mit dem jeweiligen Beschreibungs-Aktivitätswert der jeweiligen Konfigurationsbeschreibung aus der Mehrzahl von Konfigurationsbeschreibungen umfasst.

Die Vergleiche der Konfigurations-Aktivitätswerte und der Beschreibungs-Aktivitätswerte können zunächst für die erste Konfigurationsbeschreibung aus der Mehrzahl von Konfigurationsbeschreibungen und jede Konfigurationsstrategie der Mehrzahl von Konfigurationsstrategien durchgeführt werden und danach iterativ für wenigstens eine weitere Konfigurationsbeschreibung aus der Mehrzahl von Konfigurationsbeschreibungen und wiederum jede Konfigurationsstrategie der Mehrzahl von Konfigurationsstrategien durchgeführt werden. Eine solche Iteration kann beispielsweise abgebrochen werden, sobald die relevante Konfigurationsbeschreibung ermittelt wurde, ohne dass die Vergleiche für jede Konfigurationsbeschreibung aus der Mehrzahl von Konfigurationsbeschreibungen durchgeführt werden müssen.

Grundsätzlich wäre es auch möglich, dass die Vergleiche der Konfigurations-Aktivitätswerte und der Beschreibungs-Aktivitätswerte zunächst für eine erste Konfigurationsstrategie der Mehrzahl von Konfigurationsstrategien und jede Konfigurationsbeschreibung aus der Mehrzahl von Konfigurationsbeschreibungen durchgeführt werden und danach iterativ für wenigstens eine weitere Konfigurationsstrategie der Mehrzahl von Konfigurationsstrategien und wiederum jede Konfigurationsbeschreibung aus der Mehrzahl von Konfigurationsbeschreibungen durchgeführt werden.

Der Konfigurations-Aktivitätswert für eine Konfigurationsstrategie kann beispielsweise angeben, ob die Konfigurationsstrategie bei der Konfiguration des Protokolls für die radiologische Bildgebungsuntersuchung aktiv ist oder nicht. Wenn eine Konfigurationsstrategie bei der Konfiguration des Protokolls für die radiologische Bildgebungsuntersuchung aktiv ist, wird dadurch eine Anpassung der radiologischen Bildgebungsuntersuchung, insbesondere eine Anpassung von technischen Parametern der radiologischen Bildgebungsuntersuchung, entsprechend dieser Konfigurationsstrategie bewirkt.

Eine Ausführungsform sieht vor, dass jeder Strategiegruppe einer Mehrzahl von Strategiegruppen jeweils mehrere Konfigurationsstrategien aus der Mehrzahl von Konfigurationsstrategien zugeordnet sind, insbesondere mittels der Zuordnungsdaten zugeordnet sind,
- wobei das Ermitteln der relevanten Konfigurationsbeschreibung aus der Mehrzahl von Konfigurationsbeschreibungen für jede Strategiegruppe der Mehrzahl von Strategiegruppen für wenigstens eine dieser Strategiegruppe zugeordneten Konfigurationsstrategie der Mehrzahl von Konfigurationsstrategien einen Vergleich, insbesondere einen Identitätsvergleich, des jeweiligen Konfigurations-Aktivitätswerts des Konfigurationsdatensatzes mit dem jeweiligen Beschreibungs-Aktivitätswert der ersten Konfigurationsbeschreibung aus der Mehrzahl von Konfigurationsbeschreibungen umfasst.

Diejenigen Konfigurationsstrategien, welche derselben Strategiegruppe zugeordnet sind, können insbesondere sich gegenseitig ausschließende Alternativen sein und/oder denselben Aspekt der radiologischen Bildgebungsuntersuchung betreffen. Diejenigen Konfigurationsstrategien, welche derselben Strategiegruppe zugeordnet sind, können insbesondere Blättern entsprechen, welche demselben Knoten eines Entscheidungsbaums zugeordnet sind, wobei dieser Knoten der Strategiegruppe entspricht.

Die Mehrzahl von Strategiegruppen kann beispielsweise eine Kontrastmittel-Strategiegruppe umfassen, wobei die Kontrastmittel-Strategiegruppe eine erste Kontrastmittel-Konfigurationsstrategie und eine zweite Kontrastmittel-Konfigurationsstrategie aufweist. Wenn die erste Kontrastmittel-Konfigurationsstrategie bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass die radiologische Bildgebungsuntersuchung eine Kontrastmittel-Anwendung umfasst. Wenn die zweite Kontrastmittel-Konfigurationsstrategie bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass die radiologische Bildgebungsuntersuchung keine Kontrastmittel-Anwendung umfasst. Somit sind die erste Kontrastmittel-Konfigurationsstrategie und die zweite Kontrastmittel-Konfigurationsstrategie sich gegenseitig ausschließende Alternativen.

Die Mehrzahl von Strategiegruppen kann beispielsweise eine Körperform-Strategiegruppe umfassen. Insbesondere kann vorgesehen sein, dass die Körperform-Strategiegruppe eine erste Körperform-Konfigurationsstrategie, welche einer schlanken Körperform des Untersuchungsobjekts entspricht, aufweist, dass die Körperform-Strategiegruppe eine zweite Körperform-Konfigurationsstrategie, welche einer normalgewichtigen Körperform des Untersuchungsobjekts entspricht, aufweist, und dass die Körperform-Strategiegruppe eine dritte Körperform-Konfigurationsstrategie, welche einer Obesität des Untersuchungsobjekts entspricht, aufweist. Insbesondere kann vorgesehen sein, dass die erste Körperform-Konfigurationsstrategie, die zweite Körperform-Konfigurationsstrategie und die dritte Körperform-Konfigurationsstrategie sich gegenseitig ausschließende Alternativen sind.

Eine Ausführungsform sieht vor, dass die relevante Konfigurationsbeschreibung aus der Mehrzahl von Konfigurationsbeschreibungen derart ermittelt wird, dass für jede Strategiegruppe der Mehrzahl von Strategiegruppen für wenigstens eine dieser Strategiegruppe zugeordnete Konfigurationsstrategie, welche bei der Konfiguration des Protokolls für die radiologische Bildgebungsuntersuchung aktiv ist, der jeweilige Konfigurations-Aktivitätswert des Konfigurationsdatensatzes gleich dem jeweiligen Beschreibungs-Aktivitätswert der relevanten Konfigurationsbeschreibung ist.

Eine Ausführungsform sieht vor, dass für eine benutzergesteuert aktivierbare Konfigurationsstrategie der Mehrzahl von Konfigurationsstrategien eine Benutzereingabe empfangen wird, - wobei der Konfigurations-Aktivitätswert des Konfigurationsdatensatzes für die benutzergesteuert aktivierbare Konfigurationsstrategie basierend auf der Benutzereingabe generiert wird.

Die Benutzereingabe kann beispielsweise mittels einer Benutzerschnittstelle erfasst werden. Basierend auf einer das Kontrastmittel betreffenden Benutzereingabe mittels der Benutzerschnittstelle kann beispielsweise festgelegt werden, ob die radiologische Bildgebungsuntersuchung mit oder ohne Kontrastmittel-Anwendung durchgeführt wird.

Insbesondere kann vorgesehen sein, dass mittels der Benutzerschnittstelle eine Frage zur radiologischen Bildgebungsuntersuchung ausgegeben, insbesondere angezeigt, wird, die durch die Benutzereingabe beantwortet werden kann. Insbesondere können zusätzlich zu der Frage eine oder mehrere Antwortoptionen ausgegeben, insbesondere angezeigt, werden. Die Benutzereingabe kann beispielsweise eine Bestätigung der Antwortoption oder eine Auswahl einer Antwort aus den mehreren Antwortoptionen betreffen. Beispielsweise können für die Frage "Welcher Organbereich wird untersucht?" die Antwortoptionen "Thorax", "Thorax Abdomen" und "Hals Thorax Abdomen" ausgegeben werden.

Die Benutzereingabe mittels der Benutzerschnittstelle kann insbesondere nach einem Laden des Protokolls und/oder vor der Konfiguration des Protokolls erfasst werden. Insbesondere kann vorgesehen sein, dass die Benutzerschnittstelle für eine Gestensteuerung, insbesondere eine berührungsempfindliche Steuerung, eine Augensteuerung und/oder eine Sprachsteuerung eingerichtet ist. Die Benutzereingabe kann beispielsweise auf einer Geste, einem Blick und/oder einem Sprachbefehl eines Benutzers basieren.

Insbesondere kann vorgesehen sein, dass nach einer Benutzereingabe mittels der Benutzerschnittstelle, welche bewirkt, dass eine erste benutzergesteuert aktivierbare Konfigurationsstrategie bei der Konfiguration des Protokolls für die radiologische Bildgebungsuntersuchung aktiv ist, automatisch wenigstens eine zweite benutzergesteuert aktivierbare Konfigurationsstrategie, welche derselben Strategiegruppe wie die erste benutzergesteuert aktivierbare Konfigurationsstrategie zugeordnet ist und/oder welche eine ausschließende Alternative zu der ersten benutzergesteuert aktivierbaren Konfigurationsstrategie ist, bei der Konfiguration des Protokolls für die radiologische Bildgebungsuntersuchung deaktiviert ist. Dadurch können der Zeitaufwand und die Fehleranfälligkeit, die mit einem benutzergesteuerten Deaktivieren von Konfigurationsstrategien verbunden wären, vermieden werden.

Eine Ausführungsform sieht vor, dass für eine automatisch aktivierbare Konfigurationsstrategie der Mehrzahl von Konfigurationsstrategien Sensor-Messdaten empfangen werden,
- wobei der Konfigurations-Aktivitätswert des Konfigurationsdatensatzes für die automatisch aktivierbare Konfigurationsstrategie basierend auf den Sensor-Messdaten automatisch generiert wird.

Die Sensor-Messdaten können beispielsweise mittels eines Sensors erfasst werden. Der Sensor kann beispielsweise ein Elektrokardiographiegerät zum Erfassen von Elektrokardiographie-Messdaten und/oder eine Kamera, insbesondere eine 2D-Kamera und/oder eine 3D-Kamera, zum Erfassen von Kamera-Messdaten sein. Weiterhin kann vorgesehen sein, dass der Sensor das medizinische Bildgebungsgerät ist und/oder dass die Sensor-Messdaten mittels des medizinischen Bildgebungsgeräts erfasste radiologische Voruntersuchungs-Bildgebungsdaten des Untersuchungsobjekts, beispielsweise in Form eines 2D-Topogramms, sind.

Die Mehrzahl von Strategiegruppen kann beispielsweise eine Herzraten-Strategiegruppe aufweisen. Insbesondere kann vorgesehen sein, dass die Herzraten-Strategiegruppe eine automatisch aktivierbare erste Herzraten-Konfigurationsstrategie, welche einer hohen Herzrate des Untersuchungsobjekts entspricht, aufweist und dass der Konfigurations-Aktivitätswert des Konfigurationsdatensatzes für die automatisch aktivierbare erste Herzraten-Konfigurationsstrategie basierend auf Elektrokardiographie-Messdaten derart generiert wird, dass die automatisch aktivierbare erste Herzraten-Konfigurationsstrategie bei der Konfiguration des Protokolls für die radiologische Bildgebungsuntersuchung aktiv ist, wenn die Herzrate des Untersuchungsobjekts einen vorbestimmten Schwellwert überschreitet.

Insbesondere kann vorgesehen sein, dass die erste Körperform-Konfigurationsstrategie automatisch aktivierbar ist. Insbesondere kann der Konfigurations-Aktivitätswert des Konfigurationsdatensatzes für die erste Körperform-Konfigurationsstrategie basierend auf den Kamera-Messdaten derart automatisch generiert werden, dass die erste Körperform-Konfigurationsstrategie bei der Konfiguration des Protokolls für die radiologische Bildgebungsuntersuchung aktiv ist, wenn die Körperform des Untersuchungsobjekts, beispielsweise aufgrund einer entsprechenden Schwellwertunterschreitung, als schlank eingestuft wird.

Insbesondere kann vorgesehen sein, dass die zweite Körperform-Konfigurationsstrategie automatisch aktivierbar ist. Insbesondere kann der Konfigurations-Aktivitätswert des Konfigurationsdatensatzes für die zweite Körperform-Konfigurationsstrategie basierend auf Kamera-Messdaten derart automatisch generiert werden, dass die zweite Körperform-Konfigurationsstrategie bei der Konfiguration des Protokolls für die radiologische Bildgebungsuntersuchung aktiv ist, wenn die Körperform des Untersuchungsobjekts, beispielsweise aufgrund einer entsprechenden Schwellwerteinhaltung, als normalgewichtig eingestuft wird.

Insbesondere kann vorgesehen sein, dass die dritte Körperform-Konfigurationsstrategie automatisch aktivierbar. Insbesondere kann der Konfigurations-Aktivitätswert des Konfigurationsdatensatzes für die dritte Körperform-Konfigurationsstrategie basierend auf den Kamera-Messdaten derart automatisch generiert werden, dass die dritte Körperform-Konfigurationsstrategie bei der Konfiguration des Protokolls für die radiologische Bildgebungsuntersuchung aktiv ist, wenn die Körperform des Untersuchungsobjekts, beispielsweise aufgrund einer entsprechenden Schwellwertüberschreitung, als fettleibig eingestuft wird.

Eine Ausführungsform sieht vor, dass die Mehrzahl von Konfigurationsstrategien eine körperbereichsspezifische Konfigurationsstrategie, welche einen anatomischen Körperbereich betrifft, aufweist,
- wobei die Konfiguration des Protokolls eine Anpassung der radiologischen Bildgebungsuntersuchung, insbesondere eine Anpassung eines Untersuchungsbereichs der radiologischen Bildgebungsuntersuchung, in Bezug auf den anatomischen Körperbereich bewirkt, wenn die körperbereichsspezifische Konfigurationsstrategie bei der Konfiguration des Protokolls für die radiologische Bildgebungsuntersuchung aktiv ist.

Insbesondere kann vorgesehen sein, dass die körperbereichsspezifische Konfigurationsstrategie benutzergesteuert aktivierbar oder automatisch aktivierbar ist.

Eine Ausführungsform sieht vor, dass die Mehrzahl von Konfigurationsstrategien eine untersuchungsobjektspezifische Konfigurationsstrategie, welche eine Eigenschaft des Untersuchungsobjekts betrifft, aufweist, - wobei die Konfiguration des Protokolls eine Anpassung der radiologischen Bildgebungsuntersuchung in Bezug auf die Eigenschaft des Untersuchungsobjekts bewirkt, wenn die untersuchungsobjektspezifische Konfigurationsstrategie bei der Konfiguration des Protokolls für die radiologische Bildgebungsuntersuchung aktiv ist.

Das Untersuchungsobjekt kann beispielsweise ein Mensch, ein Tier oder ein Untersuchungsphantom sein. Die Eigenschaft des Untersuchungsobjekt kann beispielsweise eine Herzrate und/oder eine Körperform betreffen. Insbesondere kann vorgesehen sein, dass die untersuchungsobjektspezifische Konfigurationsstrategie benutzergesteuert aktivierbar oder automatisch aktivierbar ist.

Eine Ausführungsform sieht vor, dass durch jede Konfigurationsbeschreibung der Mehrzahl von Konfigurationsbeschreibungen jeweils eine Konfigurationsvariantenmenge, welche genau einen potentiellen Konfigurationsdatensatz oder mehrere potentielle Konfigurationsdatensätze umfasst, definiert ist,
- wobei die relevante Konfigurationsbeschreibung aus der Mehrzahl von Konfigurationsbeschreibungen derart ermittelt wird, dass der Konfigurationsdatensatz in derjenigen Konfigurationsvariantenmenge, welche durch die relevante Konfigurationsbeschreibung definiert ist, enthalten ist.

Eine Ausführungsform sieht vor, dass die Mehrzahl von Konfigurationsbeschreibungen derart hierarchisch nach Rängen sortiert ist, dass dann, wenn eine durch eine spezifischere Konfigurationsbeschreibung definierte Konfigurationsvariantenmenge eine Teilmenge einer durch eine allgemeinere Konfigurationsbeschreibung definierten Konfigurationsvariantenmenge ist, die allgemeinere Konfigurationsbeschreibung einen höheren Rang als die spezifischere Konfigurationsbeschreibung hat.

Eine Ausführungsform sieht vor, dass aus der Mehrzahl von Konfigurationsbeschreibungen ein Satz von Kandidaten-Konfigurationsbeschreibungen derart ermittelt wird, dass jede Kandidaten-Konfigurationsbeschreibung des Satzes von Kandidaten-Konfigurationsbeschreibungen ein Auswahlkriterium, welches auf dem Konfigurationsdatensatz basiert, erfüllt,
- wobei aus dem Satz von Kandidaten-Konfigurationsbeschreibungen diejenige mit dem höchsten Rang als die relevante Konfigurationsbeschreibung ermittelt wird.

Eine Ausführungsform sieht vor, dass für jede Konfigurationsbeschreibung der Mehrzahl von Konfigurationsbeschreibungen gilt, dass das Auswahlkriterium für diese Konfigurationsbeschreibung genau dann erfüllt ist, wenn der Konfigurationsdatensatz in derjenigen Konfigurationsvariantenmenge, welche durch diese Konfigurationsbeschreibung definiert ist, enthalten ist.

Insbesondere kann vorgesehen sein, dass für jede Konfigurationsbeschreibung der Mehrzahl von Konfigurationsbeschreibungen gilt, dass das Auswahlkriterium für diese Konfigurationsbeschreibung genau dann erfüllt ist, wenn für jede Strategiegruppe der Mehrzahl von Strategiegruppen für wenigstens eine dieser Strategiegruppe zugeordnete Konfigurationsstrategie, welche bei der Konfiguration des Protokolls für die radiologische Bildgebungsuntersuchung aktiv ist, der jeweilige Konfigurations-Aktivitätswert des Konfigurationsdatensatzes gleich dem jeweiligen Beschreibungs-Aktivitätswert dieser Konfigurationsbeschreibung ist.

Die Erfindung betrifft ferner ein Datenverarbeitungssystem zum Bereitstellen einer Studienbeschreibung für eine radiologische Bildgebungsuntersuchung, wobei das Datenverarbeitungssystem zum Ausführen des erfindungsgemäßen Verfahrens eingerichtet ist.

Das Datenverarbeitungssystem kann insbesondere eine Datenschnittstelle aufweisen, um Daten zu empfangen und/oder bereitzustellen. Das Datenverarbeitungssystem kann insbesondere einen Datenprozessor aufweisen, um Daten zu verarbeiten, insbesondere zu ermitteln, zu berechnen und/oder zu generieren. Der Datenprozessor kann beispielsweise mittels der Datenschnittstelle mit einem Datenspeicher verbunden sein. Die Datenschnittstelle kann beispielsweise zum Schreiben von Daten in den Datenspeicher und/oder zum Einlesen von Daten aus dem Datenspeicher eingerichtet sein.

Die Erfindung betrifft ferner ein medizinisches Bildgebungsgerät, aufweisend das erfindungsgemäße Datenverarbeitungssystem und eingerichtet zum Durchführen der radiologischen Bildgebungsuntersuchung.

Das medizinische Bildgebungsgerät kann beispielsweise eine Benutzerschnittstelle zum Erfassen einer Benutzereingabe für eine benutzergesteuert aktivierbare Konfigurationsstrategie der Mehrzahl von Konfigurationsstrategien aufweisen. Die Benutzerschnittstelle kann insbesondere zum Bereitstellen der Benutzereingabe an das Datenverarbeitungssystem eingerichtet sein.

Das medizinische Bildgebungsgerät kann beispielsweise einen Sensor zum Erfassen von Sensor-Messdaten für eine automatisch aktivierbare Konfigurationsstrategie der Mehrzahl von Konfigurationsstrategien aufweisen. Der Sensor kann insbesondere zum Bereitstellen der Sensor-Messdaten an das Datenverarbeitungssystem eingerichtet sein.

Das medizinische Bildgebungsgerät kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus, insbesondere einem PET-CT-Gerät und einem PET-MR-Gerät, besteht. Das medizinische Bildgebungsgerät kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen.

Die Erfindung betrifft ferner ein Computerprogrammprodukt, umfassend Befehle, welche einen Computer dazu veranlassen, das erfindungsgemäße Verfahren auszuführen, wenn die Befehle von dem Computer ausgeführt werden.

Die Erfindung betrifft ferner ein computerlesbares Medium, umfassend Befehle, welche einen Computer dazu veranlassen, das erfindungsgemäße Verfahren auszuführen, wenn die Befehle von dem Computer ausgeführt werden.

Das Computerprogrammprodukt kann beispielsweise ein Computerprogramm sein oder neben dem Computerprogramm mindestens einen zusätzlichen Bestandteil umfassen. Der mindestens eine zusätzliche Bestandteil des Computerprogrammprodukts kann als Hardware und/oder als Software ausgebildet sein.

Das Computerprogrammprodukt kann beispielsweise ein Speichermedium, auf dem zumindest ein Teil des Computerprogrammprodukts gespeichert ist, und/oder ein Schlüssel zur Authentifizierung eines Benutzers des Computerprogrammprodukts, insbesondere in Form eines Dongles, aufweisen. Das Computerprogrammprodukt und/oder das Computerprogramm kann beispielsweise ein Cloud-Anwendungs-Programm aufweisen, welches zum Verteilen der Befehle auf verschiedene Verarbeitungseinheiten, insbesondere verschiedene Computer, eines Cloud-Computing-Systems ausgebildet ist, wobei jede der Verarbeitungseinheiten zum Ausführen eines oder mehrerer der Befehle ausgebildet ist.

Auf dem computerlesbaren Medium kann beispielsweise das Computerprogrammprodukt nach einer der Ausführungsformen, die in dieser Anmeldung offenbart sind, und/oder das Computerprogramm nach einer der Ausführungsformen, die in dieser Anmeldung offenbart sind, gespeichert sein. Das computerlesbare Medium kann beispielsweise ein Memorystick, eine Festplatte oder ein sonstiger Datenträger sein, der insbesondere lösbar mit einem Computer verbunden oder fest in einen Computer integriert sein kann. Das computerlesbare Medium kann beispielsweise einen Bereich eines Speichersystems bilden.

Das Datenverarbeitungssystem kann beispielsweise eine oder mehrere Komponenten in Form von Hardware und/oder eine oder mehrere Komponenten in Form von Software aufweisen. Das Datenverarbeitungssystem kann beispielsweise zumindest teilweise von einem Computer und/oder von einem Cloud-Computing-System gebildet sein. Das Datenverarbeitungssystem kann beispielsweise ein Cloud-Computing-System, ein Computernetzwerk, einen Computer, einen Tabletcomputer, ein Smartphone oder ähnliches oder eine Kombination davon aufweisen.

Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Field-programmable gate array), ein ASIC-System (Application-specific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen.

Datenverarbeitungsschritte des Verfahrens können beispielsweise in dem Datenprozessor ausgeführt werden, insbesondere in Form von Berechnungen ausgeführt werden. Eine Berechnung kann insbesondere erfolgen, indem ein Algorithmus, beispielsweise ein trainierter Maschinenlernalgorithmus, auf diejenigen Daten, auf denen die Berechnung basiert, angewendet wird.

Daten, insbesondere der Konfigurationsdatensatz, die Zuordnungsdaten und/oder die Sensor-Messdaten, können beispielsweise empfangen werden, indem ein Signal, welches die Daten trägt, empfangen wird und/oder indem die Daten eingelesen werden, insbesondere aus einem Datenspeicher eingelesen werden. Daten, insbesondere die Studienbeschreibung und/oder das Bildgebungsdatenpaket, können beispielsweise bereitgestellt werden, indem ein Signal, welches die Daten trägt, übertragen wird und/oder indem die Daten in einen Datenspeicher geschrieben werden und/oder indem die Daten auf einem Bildschirm angezeigt werden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der ein System betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung des unbestimmten Artikels "ein" bzw. "eine" schließt nicht aus, dass das betroffene Merkmal auch mehrfach vorhanden sein kann. Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal berechnet (alternativ: ermittelt, generiert etc.) wird, nicht aus, dass das erste Merkmal ferner basierend auf einem dritten Merkmal berechnet (alternativ: ermittelt, generiert etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.
Die Fig. 1 zeigt eine Darstellung eines Konfigurationsdatensatzes und von Zuordnungsdaten gemäß einem ersten Beispiel.
Die Fig. 2 zeigt eine Darstellung eines Konfigurationsdatensatzes und von Zuordnungsdaten gemäß einem zweiten Beispiel.
Die Fig. 3 zeigt ein Ablaufdiagramm eines Verfahrens zum Bereitstellen einer Studienbeschreibung für eine radiologische Bildgebungsuntersuchung.
Die Fig. 4 zeigt ein Datenverarbeitungssystem.

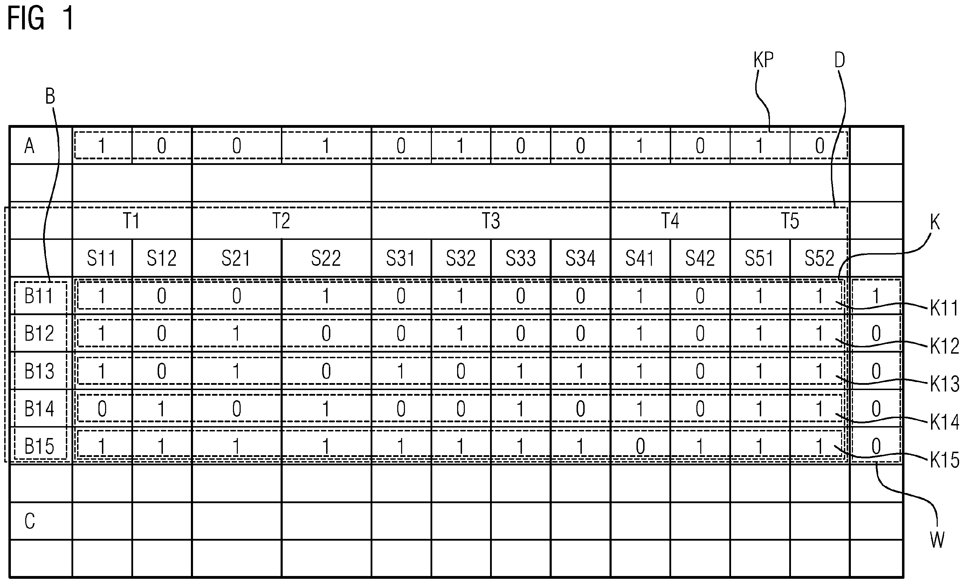

Die Fig. 1 zeigt eine Darstellung des Konfigurationsdatensatzes KP und der Zuordnungsdaten D gemäß einem ersten Beispiel.

Jeder Konfigurationsbeschreibung der Mehrzahl von Konfigurationsbeschreibungen K ist mittels der Zuordnungsdaten D jeweils eine Studienbeschreibung aus der Mehrzahl von Studienbeschreibungen B zugeordnet. Die Mehrzahl von Konfigurationsbeschreibungen K umfasst gemäß der Fig. 1 die Konfigurationsbeschreibungen K11, K12, K13, K14 und K15. Die Mehrzahl von Studienbeschreibungen B umfasst gemäß der Fig. 1 die Studienbeschreibungen B11, B12, B13, B14 und B15. Beispielsweise kann vorgesehen sein, dass die Studienbeschreibung B11 "Kopf" lautet, dass die Studienbeschreibung B12 "Kopf Hals" lautet, dass die Studienbeschreibung B13 "Kopf Hals Thorax" lautet, dass die Studienbeschreibung B14 "Low Dose" lautet und dass die Studienbeschreibung B15 "Cardio" lautet.

In dem Feld A kann, beispielsweise in Textform, ein Hinweis angezeigt werden, dass rechts davon Konfigurations-Aktivitätswerte des Konfigurationsdatensatzes KP stehen. Das kann beispielsweise in Form einer Anzeige des Worts "Aktiv" erfolgen. In dem Feld C kann beispielsweise die Studienbeschreibung, welche der relevanten Konfigurationsbeschreibung zugeordnet ist, angezeigt werden. Gemäß dem in der Fig. 1 gezeigten Beispiel ist die Konfigurationsbeschreibung K11 die relevante Konfigurationsbeschreibung. Dieser ist die Studienbeschreibung B11 zugeordnet.

Der Konfigurationsdatensatz KP weist für jede Konfigurationsstrategie einer Mehrzahl von Konfigurationsstrategien jeweils einen Konfigurations-Aktivitätswert, welcher einen Aktivitätsstatus dieser Konfigurationsstrategie bei der Konfiguration des Protokolls für die radiologische Bildgebungsuntersuchung anzeigt, auf. Jede Konfigurationsbeschreibung der Mehrzahl von Konfigurationsbeschreibungen K weist für jede Konfigurationsstrategie der Mehrzahl von Konfigurationsstrategien jeweils einen Beschreibungs-Aktivitätswert auf. Das Ermitteln der relevanten Konfigurationsbeschreibung aus der Mehrzahl von Konfigurationsbeschreibungen K umfasst für wenigstens eine Konfigurationsstrategie der Mehrzahl von Konfigurationsstrategien einen Vergleich, insbesondere einen Identitätsvergleich, des jeweiligen Konfigurations-Aktivitätswerts des Konfigurationsdatensatzes KP mit dem jeweiligen Beschreibungs-Aktivitätswert einer ersten Konfigurationsbeschreibung aus der Mehrzahl von Konfigurationsbeschreibungen K.

Der Konfigurationsdatensatz KP beschreibt somit eine Aufteilung der Mehrzahl von Konfigurationsstrategien in eine erste Teilmenge und eine zweite Teilmenge. Die Konfigurationsstrategien der ersten Teilmenge haben jeweils einen Konfigurations-Aktivitätswert, der gleich eins ist, und sind bei der Konfiguration des Protokolls für die radiologische Bildgebungsuntersuchung aktiv. Die Konfigurationsstrategien der zweiten Teilmenge haben jeweils einen Konfigurations-Aktivitätswert, der gleich null ist, und sind bei der Konfiguration des Protokolls für die radiologische Bildgebungsuntersuchung nicht aktiv.

Die Zuordnungsdaten D weisen eine Konfigurationsdatenstruktur auf. Die relevante Konfigurationsbeschreibung wird aus der Mehrzahl von Konfigurationsbeschreibungen K basierend auf dem Konfigurationsdatensatz und der Konfigurationsdatenstruktur der Zuordnungsdaten D ermittelt. Die Konfigurationsdatenstruktur basiert insbesondere auf einer Mehrzahl von Konfigurationsstrategien und ist zu dem Konfigurationsdatensatz KP kompatibel.

Es gibt eine Mehrzahl von Strategiegruppen, welche die Strategiegruppen T1, T2, T3, T4 und T5 umfasst. Jeder Strategiegruppe der Mehrzahl von Strategiegruppen sind jeweils mehrere Konfigurationsstrategien aus der Mehrzahl von Konfigurationsstrategien zugeordnet, wobei das Ermitteln der relevanten Konfigurationsbeschreibung aus der Mehrzahl von Konfigurationsbeschreibungen K für jede Strategiegruppe der Mehrzahl von Strategiegruppen für wenigstens eine dieser Strategiegruppe zugeordneten Konfigurationsstrategie der Mehrzahl von Konfigurationsstrategien einen Vergleich, insbesondere einen Identitätsvergleich, des jeweiligen Konfigurations-Aktivitätswerts des Konfigurationsdatensatzes KP mit dem jeweiligen Beschreibungs-Aktivitätswert der ersten Konfigurationsbeschreibung aus der Mehrzahl von Konfigurationsbeschreibungen K umfasst.

Die Strategiegruppe T1 betrifft den Kopf des Untersuchungsobjekts als anatomischen Körperbereich. Der Strategiegruppe T1 sind die Konfigurationsstrategie S11 und die Konfigurationsstrategie S12 zugeordnet, die jeweils körperbereichsspezifisch sind. Wenn die Konfigurationsstrategie S11 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Kopf des Untersuchungsobjekts in dem Untersuchungsbereich der radiologischen Bildgebungsuntersuchung enthalten ist. Wenn die Konfigurationsstrategie S12 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Kopf des Untersuchungsobjekts in dem Untersuchungsbereich der radiologischen Bildgebungsuntersuchung nicht enthalten ist.

Die Strategiegruppe T2 betrifft den Hals des Untersuchungsobjekts als anatomischen Körperbereich. Der Strategiegruppe T2 sind die Konfigurationsstrategie S21 und die Konfigurationsstrategie S22 zugeordnet, die jeweils körperbereichsspezifisch sind. Wenn die Konfigurationsstrategie S21 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Hals des Untersuchungsobjekts in dem Untersuchungsbereich der radiologischen Bildgebungsuntersuchung enthalten ist. Wenn die Konfigurationsstrategie S22 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Hals des Untersuchungsobjekts in dem Untersuchungsbereich der radiologischen Bildgebungsuntersuchung nicht enthalten ist.

Die Strategiegruppe T3 betrifft den Thorax des Untersuchungsobjekts als anatomischen Körperbereich. Der Strategiegruppe T3 sind die Konfigurationsstrategien S31, S32, S33 und S34 zugeordnet, die jeweils körperbereichsspezifisch sind. Wenn die Konfigurationsstrategie S31 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Thorax des Untersuchungsobjekts in dem Untersuchungsbereich der radiologischen Bildgebungsuntersuchung enthalten ist. Wenn die Konfigurationsstrategie S32 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Thorax des Untersuchungsobjekts in dem Untersuchungsbereich der radiologischen Bildgebungsuntersuchung nicht enthalten ist.

Wenn die Konfigurationsstrategie S33 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Thorax des Untersuchungsobjekts in dem Untersuchungsbereich der radiologischen Bildgebungsuntersuchung enthalten ist und dass die radiologische Bildgebungsuntersuchung für eine besonders niedrige Strahlendosis optimiert ist. Wenn die Konfigurationsstrategie S34 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass der Thorax des Untersuchungsobjekts in dem Untersuchungsbereich der radiologischen Bildgebungsuntersuchung enthalten ist und dass die radiologische Bildgebungsuntersuchung keine Kontrastmittel-Anwendung umfasst.

Die Strategiegruppe T4 betrifft die Körperform des Untersuchungsobjekts als eine Eigenschaft des Untersuchungsobjekts. Der Strategiegruppe T4 sind die Konfigurationsstrategie S41 und die Konfigurationsstrategie S42 zugeordnet, die jeweils untersuchungsobjektspezifisch sind. Wenn die Konfigurationsstrategie S41 bei der Konfiguration des Protokolls für die radiologische Bildgebungsuntersuchung aktiv ist, bewirkt die Konfiguration des Protokolls eine Anpassung der radiologischen Bildgebungsuntersuchung an eine schlanke Körperform des Untersuchungsobjekts. Wenn die Konfigurationsstrategie S42 bei der Konfiguration des Protokolls für die radiologische Bildgebungsuntersuchung aktiv ist, bewirkt die Konfiguration des Protokolls eine Anpassung der radiologischen Bildgebungsuntersuchung an eine fettleibige Körperform des Untersuchungsobjekts.

Die Strategiegruppe T5 betrifft eine iterative Metallartefaktreduktion als einen Teilprozess der radiologischen Bildgebungsuntersuchung. Der Strategiegruppe T5 sind die Konfigurationsstrategie S51 und die Konfigurationsstrategie S52 zugeordnet. Wenn die Konfigurationsstrategie S51 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass die radiologische Bildgebungsuntersuchung eine iterative Metallartefaktreduktion umfasst. Wenn die Konfigurationsstrategie S52 bei der Konfiguration des Protokolls für die radiologische Bildgebungsuntersuchung aktiv ist, bewirkt die Konfiguration des Protokolls eine Anpassung der radiologischen Bildgebungsuntersuchung derart, dass die radiologische Bildgebungsuntersuchung keine iterative Metallartefaktreduktion umfasst.

Die relevante Konfigurationsbeschreibung wird aus der Mehrzahl von Konfigurationsbeschreibungen K derart ermittelt, dass für jede Strategiegruppe der Mehrzahl von Strategiegruppen für wenigstens eine dieser Strategiegruppe zugeordnete Konfigurationsstrategie, welche bei der Konfiguration des Protokolls für die radiologische Bildgebungsuntersuchung aktiv ist, der jeweilige Konfigurations-Aktivitätswert des Konfigurationsdatensatzes KP gleich dem jeweiligen Beschreibungs-Aktivitätswert der relevanten Konfigurationsbeschreibung ist.

Durch jede Konfigurationsbeschreibung der Mehrzahl von Konfigurationsbeschreibungen K ist jeweils eine Konfigurationsvariantenmenge, welche mehrere potentielle Konfigurationsdatensätze umfasst, definiert. Die relevante Konfigurationsbeschreibung wird aus der Mehrzahl von Konfigurationsbeschreibungen K derart ermittelt, dass der Konfigurationsdatensatz KP in derjenigen Konfigurationsvariantenmenge, welche durch die relevante Konfigurationsbeschreibung definiert ist, enthalten ist.

Beispielsweise ist durch die Konfigurationsbeschreibung K11 eine Konfigurationsvariantenmenge definiert, welche zwei potentielle Konfigurationsdatensätze umfasst. Einer davon ist der Konfigurationsdatensatz KP. Der andere potentielle Konfigurationsdatensatz ist mit Ausnahme der Konfigurationsstrategien S51 und S52 identisch zu dem Konfigurationsdatensatz KP, weist für die Konfigurationsstrategie S51 einen Konfigurations-Aktivitätswert, der gleich null ist, auf und weist für die Konfigurationsstrategie S52 einen Konfigurations-Aktivitätswert, der gleich eins ist, auf.

Die Mehrzahl von Konfigurationsbeschreibungen K ist derart hierarchisch nach Rängen sortiert, dass dann, wenn eine durch eine spezifischere Konfigurationsbeschreibung definierte Konfigurationsvariantenmenge eine Teilmenge einer durch eine allgemeinere Konfigurationsbeschreibung definierten Konfigurationsvariantenmenge ist, die allgemeinere Konfigurationsbeschreibung einen höheren Rang als die spezifischere Konfigurationsbeschreibung hat. In der Darstellung, die in der Fig. 1 gezeigt ist, sind die Konfigurationsbeschreibungen derart angeordnet, dass der jeweilige Rang von oben nach unten kleiner wird.

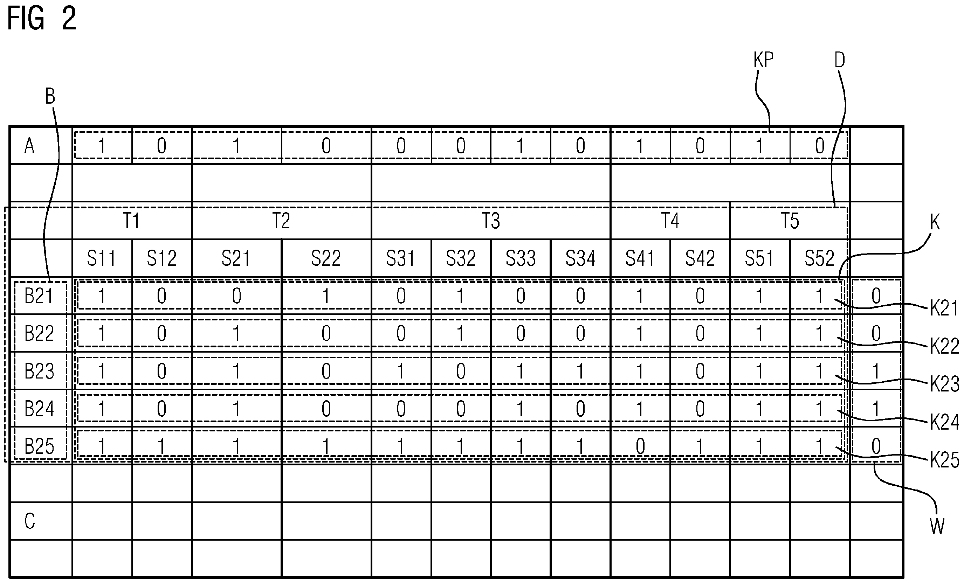

Die Fig. 2 zeigt eine Darstellung des Konfigurationsdatensatzes KP und der Zuordnungsdaten D gemäß einem zweiten Beispiel. Die Mehrzahl von Konfigurationsbeschreibungen K umfasst gemäß der Fig. 2 die Konfigurationsbeschreibungen K21, K22, K23, K24 und K25. Die Mehrzahl von Studienbeschreibungen B umfasst gemäß der Fig. 2 die Studienbeschreibungen B21, B22, B23, B24 und B25. Beispielsweise kann vorgesehen sein, dass die Studienbeschreibung B21 "Kopf" lautet, dass die Studienbeschreibung B22 "Kopf Hals" lautet, dass die Studienbeschreibung B23 "Kopf Hals Thorax" lautet, dass die Studienbeschreibung B24 "Kopf Hals Thorax Low Dose" lautet und dass die Studienbeschreibung B25 "Cardio" lautet.

Aus der Mehrzahl von Konfigurationsbeschreibungen K wird ein Satz von Kandidaten-Konfigurationsbeschreibungen derart ermittelt, dass jede Kandidaten-Konfigurationsbeschreibung des Satzes von Kandidaten-Konfigurationsbeschreibungen ein Auswahlkriterium, welches auf dem Konfigurationsdatensatz KP basiert, erfüllt, wobei aus dem Satz von Kandidaten-Konfigurationsbeschreibungen diejenige mit dem höchsten Rang als die relevante Konfigurationsbeschreibung ermittelt wird.

Für jede Konfigurationsbeschreibung der Mehrzahl von Konfigurationsbeschreibungen K gilt, dass das Auswahlkriterium für diese Konfigurationsbeschreibung genau dann erfüllt ist, wenn der Konfigurationsdatensatz KP in derjenigen Konfigurationsvariantenmenge, welche durch diese Konfigurationsbeschreibung definiert ist, enthalten ist. Für jede Konfigurationsbeschreibung der Mehrzahl von Konfigurationsbeschreibungen K gilt, dass der Konfigurationsdatensatz KP in derjenigen Konfigurationsvariantenmenge, welche durch diese Konfigurationsbeschreibung definiert ist, genau dann enthalten ist, wenn für jede Strategiegruppe der Mehrzahl von Strategiegruppen für wenigstens eine dieser Strategiegruppe zugeordnete Konfigurationsstrategie, welche bei der Konfiguration des Protokolls für die radiologische Bildgebungsuntersuchung aktiv ist, der jeweilige Konfigurations-Aktivitätswert des Konfigurationsdatensatzes gleich dem jeweiligen Beschreibungs-Aktivitätswert dieser Konfigurationsbeschreibung ist.

Jeder Konfigurationsbeschreibung der Mehrzahl von Konfigurationsbeschreibungen K ist ein Eignungswert aus der Mehrzahl der Eignungswerte W zugeordnet. Der Eignungswert ist gleich eins, wenn der Konfigurationsdatensatz in derjenigen Konfigurationsvariantenmenge, welche durch die jeweilige Konfigurationsbeschreibung definiert ist, enthalten ist. Der Eignungswert ist gleich null, wenn der Konfigurationsdatensatz in derjenigen Konfigurationsvariantenmenge, welche durch die jeweilige Konfigurationsbeschreibung definiert ist, nicht enthalten ist. Diejenigen Konfigurationsbeschreibungen, denen ein Eignungswert, der gleich eins ist, zugeordnet ist, erfüllen somit das Auswahlkriterium.

Die Konfigurationsbeschreibung K23 und die Konfigurationsbeschreibung K24 sind die einzigen Konfigurationsbeschreibungen, welche das Auswahlkriterium erfüllen. Die Konfigurationsbeschreibung K23 hat einen höheren Rang als die Konfigurationsbeschreibung K24 und ist damit die relevante Konfigurationsbeschreibung.

In dem Feld C kann beispielsweise die Studienbeschreibung, welche der relevanten Konfigurationsbeschreibung zugeordnet ist, angezeigt werden. Gemäß dem in der Fig. 2 gezeigten Beispiel ist die Konfigurationsbeschreibung K23 die relevante Konfigurationsbeschreibung. Dieser ist die Studienbeschreibung B23 zugeordnet.

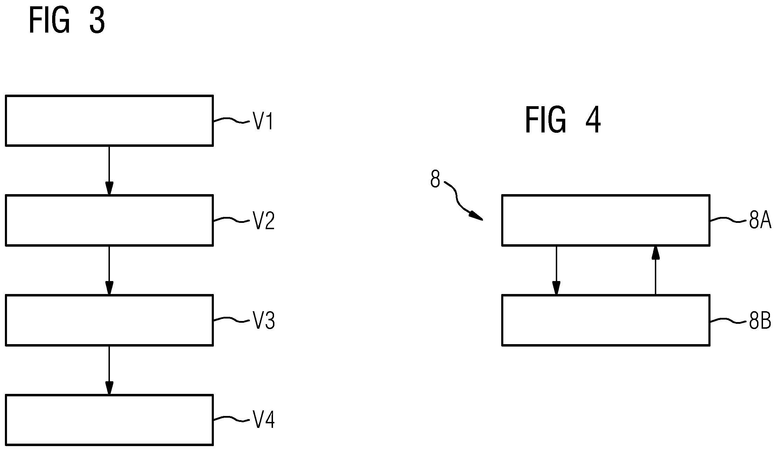

Die Fig. 3 zeigt ein Ablaufdiagramm eines Verfahrens zum Bereitstellen einer Studienbeschreibung für eine radiologische Bildgebungsuntersuchung, das Verfahren umfassend:
- ein Empfangen V1 eines Konfigurationsdatensatzes KP, welcher eine Konfiguration eines Protokolls für die radiologische Bildgebungsuntersuchung betrifft,
- ein Empfangen V2 von Zuordnungsdaten D, wobei jeder Konfigurationsbeschreibung einer Mehrzahl von Konfigurationsbeschreibungen K mittels der Zuordnungsdaten D jeweils eine Studienbeschreibung aus einer Mehrzahl von Studienbeschreibungen B zugeordnet ist,
- ein Ermitteln V3 einer relevanten Konfigurationsbeschreibung aus der Mehrzahl von Konfigurationsbeschreibungen K basierend auf dem Konfigurationsdatensatz KP und den Zuordnungsdaten D,
- ein Bereitstellen V4 derjenigen Studienbeschreibung der Mehrzahl von Studienbeschreibungen B, welche mittels der Zuordnungsdaten D der relevanten Konfigurationsbeschreibung zugeordnet ist.

Die Fig. 4 zeigt das Datenverarbeitungssystem 8 zum Bereitstellen der Studienbeschreibung für die radiologische Bildgebungsuntersuchung, wobei das Datenverarbeitungssystem 8 zum Ausführen des Verfahrens gemäß dem in der Fig. 3 gezeigten Ablaufdiagramm eingerichtet ist. Das Datenverarbeitungssystem 8 weist die Datenschnittstelle 8A auf, um Daten zu empfangen und/oder bereitzustellen. Das Datenverarbeitungssystem 8 weist den Datenprozessor 8B auf, um Daten zu verarbeiten, insbesondere zu ermitteln, zu berechnen und/oder zu generieren.

## Patentansprüche

1. Computerimplementiertes Verfahren zum Bereitstellen einer Studienbeschreibung für eine radiologische Bildgebungsuntersuchung, das Verfahren umfassend:
- ein Empfangen (V1) eines Konfigurationsdatensatzes (KP), welcher eine Konfiguration eines Protokolls für die radiologische Bildgebungsuntersuchung betrifft,
- ein Empfangen (V2) von Zuordnungsdaten (D), wobei jeder Konfigurationsbeschreibung einer Mehrzahl von Konfigurationsbeschreibungen (K) mittels der Zuordnungsdaten (D) jeweils eine Studienbeschreibung aus einer Mehrzahl von Studienbeschreibungen (B) zugeordnet ist,
- ein Ermitteln (V3) einer relevanten Konfigurationsbeschreibung aus der Mehrzahl von Konfigurationsbeschreibungen (K) basierend auf dem Konfigurationsdatensatz (KP) und den Zuordnungsdaten (D),
- ein Bereitstellen (V4) derjenigen Studienbeschreibung der Mehrzahl von Studienbeschreibungen (B), welche mittels der Zuordnungsdaten (D) der relevanten Konfigurationsbeschreibung zugeordnet ist.

2. Verfahren nach Anspruch 1,
- wobei der Konfigurationsdatensatz (KP) für jede Konfigurationsstrategie einer Mehrzahl von Konfigurationsstrategien jeweils einen Konfigurations-Aktivitätswert, welcher einen Aktivitätsstatus dieser Konfigurationsstrategie bei der Konfiguration des Protokolls für die radiologische Bildgebungsuntersuchung anzeigt, aufweist,
- wobei jede Konfigurationsbeschreibung der Mehrzahl von Konfigurationsbeschreibungen (K) für jede Konfigurationsstrategie der Mehrzahl von Konfigurationsstrategien jeweils einen Beschreibungs-Aktivitätswert aufweist,
- wobei das Ermitteln der relevanten Konfigurationsbeschreibung aus der Mehrzahl von Konfigurationsbeschreibungen (K) für wenigstens eine Konfigurationsstrategie der Mehrzahl von Konfigurationsstrategien einen Vergleich des jeweiligen Konfigurations-Aktivitätswerts des Konfigurationsdatensatzes (KP) mit dem jeweiligen Beschreibungs-Aktivitätswert einer ersten Konfigurationsbeschreibung aus der Mehrzahl von Konfigurationsbeschreibungen (K) umfasst.

3. Verfahren nach Anspruch 2,
- wobei jeder Strategiegruppe einer Mehrzahl von Strategiegruppen jeweils mehrere Konfigurationsstrategien aus der Mehrzahl von Konfigurationsstrategien zugeordnet sind,
- wobei das Ermitteln der relevanten Konfigurationsbeschreibung aus der Mehrzahl von Konfigurationsbeschreibungen (K) für jede Strategiegruppe der Mehrzahl von Strategiegruppen für wenigstens eine dieser Strategiegruppe zugeordneten Konfigurationsstrategie der Mehrzahl von Konfigurationsstrategien einen Vergleich des jeweiligen Konfigurations-Aktivitätswerts des Konfigurationsdatensatzes (KP) mit dem jeweiligen Beschreibungs-Aktivitätswert der ersten Konfigurationsbeschreibung aus der Mehrzahl von Konfigurationsbeschreibungen (K) umfasst.

4. Verfahren nach Anspruch 3,
- wobei die relevante Konfigurationsbeschreibung aus der Mehrzahl von Konfigurationsbeschreibungen (K) derart ermittelt wird, dass für jede Strategiegruppe der Mehrzahl von Strategiegruppen für wenigstens eine dieser Strategiegruppe zugeordnete Konfigurationsstrategie, welche bei der Konfiguration des Protokolls für die radiologische Bildgebungsuntersuchung aktiv ist, der jeweilige Konfigurations-Aktivitätswert des Konfigurationsdatensatzes (KP) gleich dem jeweiligen Beschreibungs-Aktivitätswert der relevanten Konfigurationsbeschreibung ist.

5. Verfahren nach einem der Ansprüche 2 bis 4,
- wobei für eine benutzergesteuert aktivierbare Konfigurationsstrategie der Mehrzahl von Konfigurationsstrategien eine Benutzereingabe empfangen wird,
- wobei der Konfigurations-Aktivitätswert des Konfigurationsdatensatzes (KP) für die benutzergesteuert aktivierbare Konfigurationsstrategie basierend auf der Benutzereingabe generiert wird.

6. Verfahren nach einem der Ansprüche 2 bis 5,
- wobei für eine automatisch aktivierbare Konfigurationsstrategie der Mehrzahl von Konfigurationsstrategien Sensor-Messdaten empfangen werden,
- wobei der Konfigurations-Aktivitätswert des Konfigurationsdatensatzes (KP) für die automatisch aktivierbare Konfigurationsstrategie basierend auf den Sensor-Messdaten automatisch generiert wird.

7. Verfahren nach einem der Ansprüche 2 bis 6,
- wobei die Mehrzahl von Konfigurationsstrategien eine körperbereichsspezifische Konfigurationsstrategie, welche einen anatomischen Körperbereich betrifft, aufweist,
- wobei die Konfiguration des Protokolls eine Anpassung der radiologischen Bildgebungsuntersuchung in Bezug auf den anatomischen Körperbereich bewirkt, wenn die körperbereichsspezifische Konfigurationsstrategie bei der Konfiguration des Protokolls für die radiologische Bildgebungsuntersuchung aktiv ist.

8. Verfahren nach einem der Ansprüche 2 bis 7,
- wobei die Mehrzahl von Konfigurationsstrategien eine untersuchungsobjektspezifische Konfigurationsstrategie, welche eine Eigenschaft eines Untersuchungsobjekts betrifft, aufweist,
- wobei die Konfiguration des Protokolls eine Anpassung der radiologischen Bildgebungsuntersuchung in Bezug auf die Eigenschaft des Untersuchungsobjekts bewirkt, wenn die untersuchungsobjektspezifische Konfigurationsstrategie bei der Konfiguration des Protokolls für die radiologische Bildgebungsuntersuchung aktiv ist.

9. Verfahren nach einem der Ansprüche 1 bis 8,
- wobei durch jede Konfigurationsbeschreibung der Mehrzahl von Konfigurationsbeschreibungen (K) jeweils eine Konfigurationsvariantenmenge, welche genau einen potentiellen Konfigurationsdatensatz (KP) oder mehrere potentielle Konfigurationsdatensätze umfasst, definiert ist,
- wobei die relevante Konfigurationsbeschreibung aus der Mehrzahl von Konfigurationsbeschreibungen (K) derart ermittelt wird, dass der Konfigurationsdatensatz (KP) in derjenigen Konfigurationsvariantenmenge, welche durch die relevante Konfigurationsbeschreibung definiert ist, enthalten ist.

10. Verfahren nach Anspruch 9,
- wobei die Mehrzahl von Konfigurationsbeschreibungen (K) derart hierarchisch nach Rängen sortiert ist, dass dann, wenn eine durch eine spezifischere Konfigurationsbeschreibung definierte Konfigurationsvariantenmenge eine Teilmenge einer durch eine allgemeinere Konfigurationsbeschreibung definierten Konfigurationsvariantenmenge ist, die allgemeinere Konfigurationsbeschreibung einen höheren Rang als die spezifischere Konfigurationsbeschreibung hat.

11. Verfahren nach Anspruch 10,
- wobei aus der Mehrzahl von Konfigurationsbeschreibungen (K) ein Satz von Kandidaten-Konfigurationsbeschreibungen derart ermittelt wird, dass jede Kandidaten-Konfigurationsbeschreibung des Satzes von Kandidaten-Konfigurationsbeschreibungen ein Auswahlkriterium, welches auf dem Konfigurationsdatensatz (KP) basiert, erfüllt,
- wobei aus dem Satz von Kandidaten-Konfigurationsbeschreibungen diejenige mit dem höchsten Rang als die relevante Konfigurationsbeschreibung ermittelt wird.

12. Verfahren nach Anspruch 11,
- wobei für jede Konfigurationsbeschreibung der Mehrzahl von Konfigurationsbeschreibungen (K) gilt, dass das Auswahlkriterium für diese Konfigurationsbeschreibung genau dann erfüllt ist, wenn der Konfigurationsdatensatz (KP) in derjenigen Konfigurationsvariantenmenge, welche durch diese Konfigurationsbeschreibung definiert ist, enthalten ist.

13. Datenverarbeitungssystem (8) zum Bereitstellen einer Studienbeschreibung für eine radiologische Bildgebungsuntersuchung, wobei das Datenverarbeitungssystem (8) zum Ausführen des Verfahrens nach einem der Ansprüche 1 bis 12 eingerichtet ist.

14. Medizinisches Bildgebungsgerät, aufweisend das Datenverarbeitungssystem (8) nach Anspruch 13 und eingerichtet zum Durchführen der radiologischen Bildgebungsuntersuchung.

15. Computerprogrammprodukt oder computerlesbares Medium, umfassend Befehle, welche einen Computer dazu veranlassen, das Verfahren nach einem der Ansprüche 1 bis 12 auszuführen, wenn die Befehle von dem Computer ausgeführt werden.
